**Europäisches Patentamt**

**European Patent Office**

**Office européen des brevets**

(19)

(11) Veröffentlichungsnummer: **0 031 126**

**A1**

(12) # EUROPÄISCHE PATENTANMELDUNG

(21) Anmeldenummer: **80107937.7**

(22) Anmeldetag: **16.12.80**

(51) Int. Cl.³: **A 61 C 1/08**

(30) Priorität: **20.12.79 DE 2951411**

(43) Veröffentlichungstag der Anmeldung:
**01.07.81 Patentblatt 81/26**

(84) Benannte Vertragsstaaten:
**AT BE CH FR GB IT LI LU NL SE**

(71) Anmelder: **Himmelsbach, Richard**
**Seligenstädterstrasse 41**
**D-8757 Karlstein(DE)**

(71) Anmelder: **Pfeiffer, Hans-Jörg, Dipl.-Kfm.**
**Seewaldweg 3**
**D-8110 Seehausen/Staffelsee(DE)**

(72) Erfinder: **Himmelsbach, Richard**
**Seligenstädterstrasse 41**
**D-8757 Karlstein(DE)**

(72) Erfinder: **Pfeiffer, Hans-Jörg, Dipl.-Kfm.**
**Seewaldweg 3**
**D-8110 Seehausen/Staffelsee(DE)**

(74) Vertreter: **Melzer, Wolfgang, Dipl.-Ing.**
**Bärenmühlweg 31**
**D-8120 Weilheim i.OB(DE)**

(54) **Filtriereinrichtung zur bakteriellen Entkeimung von Kühlflüssigkeit bei kieferchirurgischen Geräten.**

(57) Die Filtriereinrichtung weist eine steril verpackbare sogenannte Wegwerf-Einheit (1) auf, die einstückig ein Kühlröhrchen (5), einen Abführschlauch (4) und ein Filter (3) einschließlich einer Filtereinlage (8) aufweist. Der Abführschlauch (4) ist zwischen Kühlröhrchen (5) und Filter (3) angeordnet und mit beiden fest verbunden, wie mittels Klebung. Zur Arbeitsvereinfachung und zur leichteren Anpassung an unterschiedliche Bauarten des kieferchirurgischen Geräts kann zumindest in die Kühlflüssigkeitsbohrung (19) eine Steckereinheit (2) eingesetzt werden, die mit dem Filter (3) direkt, wie über einen Schnellverschluß, verbindbar ist oder einstückig mit diesem verbunden ist.

Die erfindungsgemäße Filtriereinrichtung ist besonders zweckmäßig bei innengekühlten Bohr- und Fräsverorrichtungen sowie bei der zahnärztlichen Implantologie.

FIG. 1

FILTRIEREINRICHTUNG ZUR BAKTERIELLEN ENTKEIMUNG
VON KÜHLFLÜSSIGKEIT BEI KIEFERCHIRURGISCHEN
GERÄTEN

Die Erfindung betrifft eine Filtriereinrichtung zur bakteriellen Entkeimung von Kühlflüssigkeit, insbesondere Kühlwasser, bei kieferchirurgischen Geräten.

Bei kieferchirurgischen Geräten werden der zahnärztlichen Einheit, die im Allgemeinen einen sehr kleinen Elektromotor enthält und an die ein zahnärztliches Handstück ankuppelbar ist, neben Strom über Versorgungskabeln auch über gesonderte Kanäle Luft und Kühlflüssigkeit, insbesondere Kühlwasser, zugeführt, wobei an der zahnärztlichen Einheit Steckerbuchsen vorgegebener Größe und vorgegebenen Abstandes vorgesehen sind.

Insbesondere wenn an der zahnärztlichen Einheit ein zahnärztliches Handstück mit einem Bohrer oder einem Fräser angekoppelt ist, ist das Kühlen des Arbeitsgebietes von erheblicher Bedeutung, um einerseits bei der Arbeit (Bohren bzw. Fräsen) entstehende Späne zu entfernen, andererseits aber auch, um zu verhindern, daß bei der Bearbeitung durch Reibungswärme Randnekrosen auftreten, die den Heilungsprozess verlangsamen, u. U. auch verhindern können. Letzteres ist insbesondere wesentlich bei der Implantologie.

-2-

Insbesondere bei der Implantologie ist es darüber hinaus wesentlich, daß die Kühlflüssigkeit bakteriologisch keimfrei ist.

In den meisten Fällen wird eine solche keimfreie Kühlflüssigkeit als Kochsalzlösung oder als entkeimtes Wasser mittels einer Injektionsnadel zugeführt. Diese Vorgehensweise ist offensichtlich umständlich.

Zur Überwindung dieser Nachteile wurde daher schon ein kieferchirurgisches Gerät der im Oberbegriff des Anspruchs 1 dargestellten Art entwickelt. Bei diesem zahnärztlichen Gerät wird ein Steckerteil in die beiden Buchsen für Kühlflüssigkeit und Luft eingesetzt, wobei der Luftdurchtritt gesperrt ist. Über einen Silikonschlauch wird die Kühlflüssigkeit einem mittels einer Klemme an der zahnärztlichen Einheit anklemmbaren Sterilfilter zugeführt, das aufschraubbar ist und in das Scheibenfilter einlegbar sind. Abstromseitig wird ein weiterer Silikonschlauch zu einem winkeligen Kühlröhrchen geführt, dessen einer Schenkel verdickt ausgebildet ist, damit an ihm der Silikonschlauch befestigt werden kann, und dessen anderer Schenkel einen kleinen Außendurchmesser besitzt. Zwar wird mit dem Sterilfilter das aus der zahnärztlichen Einheit bezogene Wasser entkeimt und für die enossale Kühlung brauchbar gemacht, wobei Pumpeinrichtungen unnötig sind. Scheibenfilter, die eine derartige Entkeimung erreichen, sind handelsüblich und werden beispielsweise von der Firma Millipore vertrieben.

Es hat sich jedoch gezeigt, daß diese Filtriereinrichtung im Gebrauch nicht immer zuverlässig arbeitet. Das Kühlröhrchen ist nämlich für anorganische Kontamination, insbesondere Rohrverkalkung, anfällig, weshalb die Gefahr einer Verstopfung der wasserführenden Kanüle besteht. Darüber

-3-

hinaus weist die bekannte Filtriereinrichtung eine ziemlich große Bauform auf und ist daher unbequem. Schließlich ist, da Scheibenfilter eingelegt werden müssen, eine einwandfreie Sterilität der Filtriereinrichtung und damit eine sichere Entkeimung der dem Arbeitsbereich zugeführten Kühlflüssigkeit nicht gewährleistet.

Es ist daher Aufgabe der Erfindung, eine Filtriereinrichtung der eingangs genannten Art so auszubilden, daß die bakterielle Entkeimung bei kleiner Bauform gewährleistet werden kann.

Die Erfindung geht von der Erkenntnis aus, daß dies dadurch erreicht werden kann, daß zumindest alle Teile stromab des Filters als vorsterilisierbares Einmalsystem ausgebildet sind.

Die Aufgabe wird also erfindungsgemäß dadurch gelöst, daß Kühlröhrchen, Abführschlauch und Filter einschließlich Filtereinlage als einstückige Einheit ausgebildet sind.

Von besonderem Vorteil ist es dabei, wenn die Steckereinheit direkt mit dem Zuführende des Filters verbindbar ist, insbesondere mit einer Schnellverbindung, wie einer Luer-Lock-Schraubverbindung. Zwar kann es auch Vorteile bringen, wenn ein einstückiges Teil aus Steckereinheit und einstückiger Einheit gebildet ist, jedoch ist es im Allgemeinen ausreichend, eine einzige mehrfach verwendbare Steckereinheit vorzusehen, die in die Buchsen in der zahnärztlichen Einheit einsetzbar ist, wobei eine Luftzufuhr mittels einer Blindkanüle verhindert ist. Die über den Kühlflüssigkeitskanal zugeführte Kühlflüssigkeit, insbesondere Wasser, wird nämlich erst im Filter entkeimt, weshalb es ausreicht, diese zusammen mit den stromab befindlichen

-4-

Teilen, Abführschlauch und Kühlröhrchen, als einmal verwendbare Einheit auszubilden.

Die erfindungsgemäße Ausbildung erlaubt es, das Kühlröhrchen aus einem durchgehend gleichen Innen- und Außendurchmesser besitzenden Röhrchen, wie einem Stahlröhrchen, zu bilden, da eine Reinigung bezüglich der anorganischen Kontaminationen nicht mehr erforderlich ist. Dieses Kühlröhrchen ist vorzugsweise in dem einen Ende des Silikonschlauchs sterilisierbar und dicht befestigt, wie beispielsweise eingeklebt, während das andere Ende des Silikonschlauchs mit dem Abführende des Filters dicht verbunden, insbesondere verklebt ist, wobei das Gehäuse des Filters ebenfalls aus einem Kunststoff besteht.

Von besonderem Vorteil ist die erfindungsgemäße Filtriereinrichtung bei einem kieferchirurgischen Gerät, bei dem das mit dem Handstück verbindbare Werkzeug innengekühlt ist, d.h., eine Zentralbohrung aufweist, in die das abgewinkelte Ende des Kühlröhrchens einsetzbar ist, wodurch eine besondere günstige Kühlwirkung erreicht wird, durch die eine äußerst geringe Randdevitalisierung des Arbeitsbereiches auftritt, was insbesondere für die Implantologie von erheblicher Bedeutung ist. Dabei ist nahe der Halterung für das Werkzeug auch eine Halterung für das Kühlröhrchen im Handstück vorgesehen, wobei die beiden Halterungen integriert ausgebildet sein können.

Die Erfindung wird durch die Merkmale der anderen Unteransprüche vorteilhaft weitergebildet.

Die Erfindung gibt also eine als Einmalsystem ausgebildete Filtriereinrichtung an, die sehr schnell einsetzbar ist und im Einsatz sicher arbeitet, das heißt, bei der dem

-5-

Arbeitsgebiet sicher entkeimte Kühlflüssigkeit zugeführt wird.

Die Erfindung wird anhand der in der Zeichnung dargestellten Ausführungsbeispiele näher erläutert. Es zeigen

Fig. 1 schematisch eine erfindungsgemäße Filtriereinrichtung, bei der das Filter an der Steckereinheit anschraubbar ist,

Fig. 2 den Schnitt II-II in Fig. 1,

Fig. 3 im Teilschnitt ein anderes Ausführungsbeispiel der Steckereinheit gemäß Fig. 1.

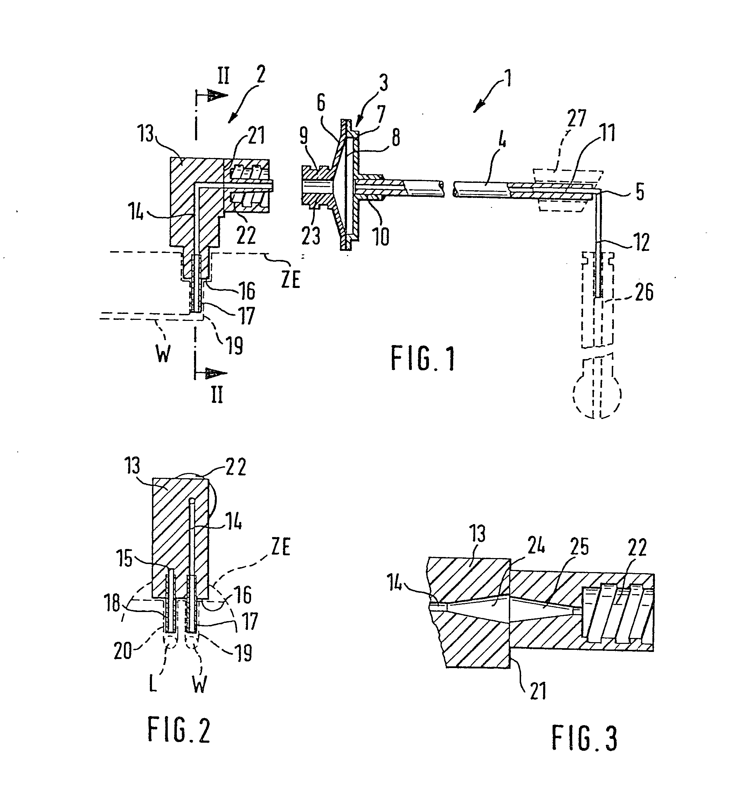

Die in Fig. 1 dargestellte Filtriereinrichtung besteht aus einer einstückigen Einheit 1 und einer Steckereinheit 2. Die einstückige Einheit besteht im wesentlichen aus einem Filter 3, einem Abführschlauch 4 und einem Kühlröhrchen 5. Das Filter 3 weist beim dargestellten Ausführungsbeispiel zwei im wesentlichen ähnlich ausgebildete Gehäuseteile 6 und 7 auf, zwischen denen eine Filtereinlage 8 dicht befestigt ist. Das erfolgt beim dargestellten Ausführungsbeispiel durch Einlegen zwischen die Umfangsränder der beiden Gehäuseteile 6, 7 und deren einstückige Verbindung miteinander, beispielsweise durch Verschweißen. Das Gehäuseteil 6 weist einen Zuführnippel 9 und das Gehäuseteil 7 einen Abführnippel 10 auf. In dem Abführnippel 10 ist der vorzugsweise aus Silikon bestehende Abführschlauch 4 sterilisierbar und dicht befestigt, insbesondere eingeklebt. Am anderen Ende des Abführschlauches 4 ist, wie dargestellt, das Kühlröhrchen 5 eingesetzt, das aus einer rechtwinkelig gebogenen und durchgehend konstanten Innen- und Außendurchmesser aufweisenden Kanüle, vorzugsweise aus Edelstahl, besteht. Auf den einen Schenkel 1 ist der Abführschlauch 4 aufgesteckt und kann beispielsweise durch Klebung fest verbunden sein. Der andere Schenkel 12 ist zur Abgabe der durch den Zuführnippel 9 zugeführten und im Filter 3 entkeimten Kühlflüssigkeit, insbesondere Kühlwasser, zum Arbeitsgebiet vorgesehen.

Die einstückige Einheit 1 ist erfindungsgemäß als vorsterilisierbare Einmaleinheit ausgebildet.

Die Steckereinheit 2 besteht beim dargestellten Ausführungsbeispiel aus einem Kunststoffkörper 13, der eine Durchgangsbohrung 14 und eine Blindbohrung 15 aufweist. In Durchgangsbohrung 14 und Blindbohrung 15 sind an dem gemeinsamen Ende 16 des Kunststoffkörpers 13 Steckerstift-Röhrchen 17 bzw. 18 eingesetzt, die ihrerseits in die Steckerbuchsen 19 bzw. 20 der zahnärztlichen Einheit ZE einsetzbar sind, die mit den Zuführkanälen für Kühlflüssigkeit (Zuführkanal W) bzw. für Luft (Zuführkanal L) verbunden sind. Dabei ist das Steckerstift-Röhrchen 18 in der Blindbohrung 15 in die Buchse 20 einsetzbar, die mit dem Zuführkanal L verbunden ist, während das in die Durchgangsbohrung 14 eingesetzte Steckerstift-Röhrchen 17 in die Buchse 19 einsetzbar ist, die mit dem Kühlflüssigkeitskanal W verbunden ist. Die zahnärztliche Einheit ZE ist in den Fig. 1 und 2 in Strichlinien angedeutet. Das andere Ende der Durchgangsbohrung 14 hat beim dargestellten Ausführungsbeispiel einen solchen Verlauf, daß dieses andere Ende bei in die Buchsen 19, 20 eingesetztem Kunststoffkörper 13 parallel zur Achse der in Strichlinien dargestellten zahnärztlichen Einheit verläuft. Mit diesem Ende 21 des Kunststoffkörpers 13 ist der "männliche" Teil 22 einer Schnellverbindung vorgesehen, deren "weiblicher" Teil 23 am Zuführnippel 9 des Filters 3 vorgesehen ist. Als solche Schnellverbindung eignet sich insbesondere eine Luer-Lock-Schraubverbindung.

Das in Fig. 3 dargestellte Ausführungsbeispiel unterscheidet sich von dem gemäß Fig. 1 nur dadurch, daß am am Ende 21 des Kunststoffkörpers 13 liegenden Endabschnitt die Durchgangsbohrung 14 eine konusförmige Aufweitung 24 besitzt, während im zugehörigen männlichen Teil 22 der Schnellverbindung eine konusförmige Verengung 25 vorgesehen ist, um besonders günstige Zuströmverhältnisse zur Filtereinlage 8 im Filter 3 zu erreichen.

In Fig. 1 sind weiter in Strichlinien Teile des zahnärztlichen Handstückes vorgesehen, das an die zahnärztliche Einheit ZE ankuppelbar ist, und an denen die erfindungsgemäße Filtriereinrichtung vorgesehen werden kann. Beim dargestellten Ausführungsbeispiel weist das Bohr- bzw. Fräswerkzeug 26 eine mittige Innenbohrung auf, in die der Schenkel 12 des Kühlröhrchens 5 einführbar ist, wobei weiter eine Halterung 27 vorgesehen ist, in die der andere Schenkel 11 des Kühlröhrchens 5 bzw. der ihn umgebende Teil des Abführschlauches 4 einlegbar ist, um so Rückstoßkräften beim Ausstoßen der Kühlflüssigkeit aus dem Kühlröhrchen 5 entgegenzuwirken. Die Vorrichtung zum Haltern des Werkzeuges 26 und die Halterung 27 können miteinander integriert ausgebildet sein.

Bei der Herstellung wird die einstückige Einheit 1 mit ETO vorsterilisiert und in eine sterile Aufreißpackung eingebracht. Dadurch wird die bisher notwendige zeitaufwendige Dampfsterilisation von Schlauch, Filter bzw. Filtergehäuse und Kühlröhrchen eingespart. Die Steckereinheit 2 ist für jedes kieferchirurgische Gerät nur einmal vorzusehen und kann mit diesem sterilisiert werden, weshalb auch statt dem Kunststoffkörper 13 ein (nicht dargestellt) Stahlkörper gleicher oder ähnlicher Ausbildung vorgesehen sein kann.

Im Gebrauch wird nach üblichem Zusammenbau der zahnärztlichen Einheit ZE und des Handstücks mit dem Werkzeug 26 und Einsetzen der Steckereinheit 2 die Aufreißpackung geöffnet und die einstückige Einheit 1 entnommen, werden männliches und weibliches Teil 22, 23 der Schnellverbindung miteinander in Eingriff gebracht und wird das Kühlröhrchen 5 mit seinem Schenkel 12 so angeordnet,daß die Austrittsöffnung des Schenkels 12 auf das Arbeitsgebiet gerichtet ist, insbesondere wird der Schenkel 12 in die Zentralbohrung des Werkzeugs 26 eingesetzt, um so eine Innenkühlung zu erreichen. Auf diese Weise wird mittels eines auf Integrität vorgetesteten Steril-Wasserfilters eine bakterielle Kontamination innerhalb des Operationsgebietes verhindert. Dadurch wird eine Infektion, die beispielsweise zum Abstoßen eines Implantates führen könnte, vermieden. Darüber hinaus wird die für anorganische Kontaminationen (Rohrverkalkung) anfällige wasserführende Kanüle bei mehrfach verwendbaren Systemen vermieden und eine zwangsweise Sterilisation der wasserführenden Teile erreicht. Durch die Minderung des anorganischen Kontaminationsrisikos wird eine einwandfreie Kühlwirkung des Werkzeuges 26, d.h., der Bohr- oder Fräsvorrichtung gewährleistet und kann, insbesondere bei Innenkühlung, eine Knochennekrose durch Überhitzung verhindert werden.

Für die erfindungsgemäße Filtriereinrichtung haben sich bei deren Anwendung für ein innengekühltes kieferchirurgisches Gerät folgende Abmessungen als zweckmäßig erwiesen: Das Kühlröhrchen ist ein gebogenes Edelstahlröhrchen mit ca. 0,75 mm Außendurchmesser und 0,2 mm Innendurchmesser mit einer Länge des Schenkels 11 von etwa 15 mm und einer Länge des Schenkels 12 von etwa 22 mm. Der Abführschlauch 4, der vorzugsweise ein PVC- oder ein Silikonschlauch ist, besitzt einen Außendurchmesser von etwa 2,3 mm und einen Innendurchmesser von ebenfalls 0,75 mm, wobei die Länge etwa 150 mm beträgt. Die sterilisierende Filtereinlage 8 besitzt vorzugsweise eine Porenweite von 0,2 μm.

Selbstverständlich sind noch andere Ausführungsbeispiele möglich, beispielsweise kann die Verbindung zwischen dem Zuführnippel 9 und der Buchse 19 auch über ein herkömmliches Steckerteil erfolgen, über dessen Austrittskanüle ein flexibler Schlauch geschoben wird, dessen anderes Ende direkt mit dem Zuführnippel 9 dicht verbindbar ist, oder auch mittels eines Doppelkonus-Verbindungsstücks.

Andererseits kann die Filtriereinrichtung auch so ausgebildet sein, daß der Kunststoffkörper 13 des Steckerteils 2 mit dem Gehäuseteil 6 des Filters 3 einstückig untrennbar verbunden ist und ebenfalls wie die einstückige Einheit 1 als Einmaleinheit ausgebildet ist. Letztere Ausbildungsform kann sich dann als besonders vorteilhaft erweisen, wenn hohe Stückzahlen zu erwarten sind, da der Mehraufwand dann durch die schnellere Handhabung gerechtfertigt erscheint.

Weiter kann bei dem in Fig. 1 und 3 dargestellten Ausführungsbeispiel der männliche Teil 22 der Schnellverbindung einteilig mit dem Kunststoffkörper 13 ausgebildet sein. Schließlich ist auch die dargestellte Form der Gehäuseteile 7, 8 des Filters 3 nicht zwingend, wesentlich ist, daß über die gesamte Fläche der Filtereinlage 8 die Entkeimung fördernde günstige Strömungsverhältnisse erreicht werden.

-10-

ANSPRÜCHE

1. Filtriereinrichtung zur bakteriellen Entkeimung von Kühlflüssigkeit, insbesondere Kühlwasser, bei kieferchirurgischen Geräten, mit einem Filter, das an der zahnärztlichen Einheit anbringbar ist und eine Filtereinlage enthält, einer Zuführleitung der Kühlflüssigkeit, die einerseits mit dem Filter und andererseits mit dem zur zahnärztlichen Einheit geführten Kühlflüssigkeitskanal verbindbar ist, und einer flexiblen Abführleitung, die von dem Filter zu einem in ihr befestigten nach Art einer Düse wirkenden Kühlröhrchen führt, über das die Kühlflüssigkeit zum Arbeitsort abgebbar ist, dadurch gekennzeichnet, daß Kühlröhrchen (5), Abführschlauch (4) und Filter (3) einschließlich Filtereinlage (8) als einstückige Einheit (1) ausgebildet sind.

2. Filtriereinrichtung nach Anspruch 1, dadurch gekennzeichnet, daß zur Verwendung als sterilisierbarer Einwegartikel, das Filter (3) der einstückigen Einheit (1) als Einmalfilter ausgebildet ist.

3. Filtriereinrichtung nach Anspruch 1 oder 2, dadurch gekennzeichnet, daß das Kühlröhrchen (5) der einstückigen Einheit (1) ein rechtwinklig gebogenes Röhrchen ist,dessen Außen- und Innendurchmesser konstant ist.

4. Filtriereinrichtung nach einem der Ansprüche 1 bis 3, gekennzeichnet durch eine in die Kühlflüssigkeitsbohrung (W, 19) direkt einsetzbare und direkt mit dem Filter (3) verbindbare Steckereinheit (2).

5. Filtriereinrichtung nach Anspruch 4, dadurch gekennzeichnet, daß zur Lagestabilisierung die Steckereinheit (2) mittels eines Blindröhrchens (18) auch in den Luftkanal (L, 20) der zahnärztlichen Einheit (ZE) einsetzbar ist.

6. Filtriereinrichtung nach Anspruch 4 oder 5, gekennzeichnet durch eine Schnellverbindung (22, 23) zwischen Steckereinheit (2) und einstückiger Einheit (1), insbesondere einer Luer-Lock-Schraubverbindung.

7. Filtriereinrichtung nach Anspruch 6, dadurch gekennzeichnet, daß an der Steckereinheit (2) der männliche Teil (22) der Schnellverbindung vorgesehen ist.

8. Filtriereinrichtung nach Anspruch 4 oder 5, dadurch gekennzeichnet, daß einstückige Einheit (1) und Steckereinheit (2) zusammen selbst als sterilisierbares einstückiges Teil ausgebildet sind.

-12-

9. Filtriereinrichtung nach einem der Ansprüche
   1 bis 8,
   gekennzeichnet durch deren Anwendung bei einer
   innengekühlten Bohr- oder Fräsvorrichtung (26, 27),
   bei der eine Halterung (27) des Kühlröhrchens (5)
   am zahnärztlichen Handstück vorgesehen ist.


10. Filtriereinrichtung nach einem der Ansprüche
    1 bis 9,
    gekennzeichnet durch deren Anwendung bei der zahn-
    ärztlichen Implantologie.

FIG.1

FIG.2

FIG.3

Europäisches
Patentamt

**EUROPÄISCHER RECHERCHENBERICHT**

| | EINSCHLÄGIGE DOKUMENTE | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| A | GB - A - 1 498 740 (SVEN-GUNNAR HESSELGREN) <br> * Ansprüche * <br><br> -- | 1 | A 61 C 1/08 |
| A | FR - A - 2 325 353 (SIEMENS) <br> * Seite 6, Zeilen 30 bis 40; Seite 7, Zeilen 1 bis 4; Figur 4a * <br><br> -- | 5 | |
| A | US - A - 4 021 920 (KIRSCHNER) <br> * Spalte 3, Zeilen 47 bis 68; Figur 1 * <br><br> ----- | 3 | RECHERCHIERTE SACHGEBIETE (Int. Cl.³) <br><br> A 61 C <br> A 61 L <br> B 01 D |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 27.03.1981 | VANRUNXT |

EPA form 1503.1  06.78